# EUROPÄISCHE PATENTSCHRIFT

(11) **EP 1 250 113 B1**
(45) Veröffentlichungstag und Bekanntmachung des Hinweises auf die Patenterteilung: **21.04.2004**
(21) Anmeldenummer: 01900352.4
(22) Anmeldetag: 18.01.2001
(51) Int. Cl.: A61F 13/26

(54) **TAMPON-APPLIKATOR**
TAMPON APPLICATOR
APPLICATEUR DE TAMPON

(30) Priorität: 18.01.2000 CH 89002000
(43) Veröffentlichungstag der Anmeldung: 23.10.2002
(73) Patentinhaber: Cimber, Hugo, 3005 Bern (CH)
(72) Erfinder: Cimber, Hugo, 3005 Bern (CH)
(74) Vertreter: BOVARD AG - Patentanwälte
(86) Internationale Anmeldenummer: PCT/CH2001/000040
(87) Internationale Veröffentlichungsnummer: WO 2001/052785

(56) Entgegenhaltungen:
- DE-A- 3 248 152
- GB-A- 753 294
- GB-A- 2 277 447
- US-A- 2 178 840
- US-A- 3 683 915
- US-A- 4 318 405
- US-A- 4 335 720
- US-A- 5 676 647

## Beschreibung

Die vorliegende Erfindung bezieht sich auf einen Tampon-Applikator gemäss dem Oberbegriff des Patentanspruchs 1.

Die Anwendung von Tampons mit einem medikamentösen Mittel werden insbesondere angewendet, um eine pH-Verschiebung im Scheidenmilieu in den alkalischen Bereich und somit eine verminderte Infektionsabwehr zu vermeiden. Hierfür werden als medikamentöse Mittel insbesondere Milchsäure- bzw. Lactobazillen- enthaltende Präparate verwendet.

Derartige Tampon-Applikatoren sind bekannt. So zeigt beispielsweise die EP-A-0 749 741 eine entsprechende Einrichtung, bei welcher vor dem Tamponkopf in der Einführhülse eine geschlossene Kammer angeordnet werden kann, was beispielsweise durch Aufstecken oder Aufschrauben eines Zusatzbehälters erfolgt, welcher mit dem entsprechenden medikamentösen Mittel, das in flüssiger Form vorliegt, gefüllt ist. Die durch den Behälter gebildete Kammer ist gegen die Einführhülse hin mit einer Folie abgeschlossen, während die andere Seite mit einem abnehmbaren Deckel versehen ist.

Vor dem Einführen oder beim Einführen des Tampons in die Vagina soll das in der Kammer enthaltene medikamentöse Mittel mit dem in der Einführhülse sich befindenden Tampon in Kontakt kommen. Die dem Tamponkopf zugewandte Folie des Behälters muss aufgebrochen werden, was durch Vorschieben des Tampons durch die Ausstosshülse erfolgen soll, beispielsweise während des Einführens des Tampons. Zur Erleichterung des Aufbrechens der Folie sind mechanische Mittel vorgesehen.

Von Nachteil ist bei dieser Einrichtung, dass das medikamentöse Mittel in einem zusätzlichen verschlossenen Behälter untergebracht ist, der vor der Anwendung auf die Einführhülse aufgesetzt werden muss. Hierzu müssen die entsprechenden Verbindungsmittel sowohl am Behälter als auch an der Einführhülse vorgesehen sein. Dadurch wird das Herstellen des Behälters und das Abfüllen des medikamentösen Mittels mit den entsprechenden Abschlüssen relativ aufwendig und entsprechend teuer. Das selbe gilt auch für die Einführhülse, die für die Aufnahme des Behälters angepasst sein muss.

Ferner zeigt beispielsweise die US-A-4 318 405 einen Tampon-Applikator, bei welchem in der Spitze des Tampons eine zylindrische Vertiefung angebracht ist, in welche eine Kapsel eingesetzt ist, die das medikamentöse Mittel enthält. Damit diese Kapsel nicht herausfallen kann, ist vorgesehen, dass diese mit einer haftenden Substanz im Tampon befestigt ist.

Dieser Tampon-Applikator weist den Nachteil auf, dass durch das Anbringen der Vertiefung im Tampon und das Einsetzen der Kapsel in dieser Vertiefung, insbesondere wenn eine haftende Substanz verwendet wird, die Herstellung aufwendig wird. Bei maschineller Fertigung sind hierzu komplizierte Aggregate erforderlich.

Die Aufgabe der vorstehenden Erfindung besteht nun darin, einen Tampon-Applikator zu schaffen, bei welchem die vorgenannten Nachteile ausgeräumt sind, welcher ein einfaches und günstiges Produzieren der einzelnen Bestandteile und ein einfaches Zusammensetzen ermöglicht, und welcher in einfacher Form anwendbar ist.

Erfindungsgemäss erfolgt die Lösung dieser Aufgabe durch die im Anspruch 1 aufgeführten Merkmale.

Durch die Möglichkeit, dass das medikamentöse Mittel in Pillenform hergestellt werden kann, welche Pille zwischen Einführhülse und Tampon eingesetzt werden kann, wird die Herstellung und die Anwendung des Tampon-Applikators wesentlich erleichtert. Die Pille, die durch Pressen eines pulverförmigen Präparats erhalten werden kann, enthält beispielsweise die Milchsäure bzw. die Lactobazillen. Die so geformte Pille löst sich dann in der Vagina auf und die medikamentösen Mittel können ihre Wirkung voll entfalten.

Ein Vorteil der Erfindung besteht darin, dass die Pille derart in der Einführhülse untergebracht ist, dass sie zwischen die Spitze des Tampons und das einführseitige Ende der Einführhülse zu liegen kommt. Dadurch kann ein einfaches Zusammensetzen des Tampon-Applikators erreicht werden, wobei insbesondere ein üblicherweise verwendbarer Tampon unverändert verwendet werden kann. Hierzu kann die Einführhülse mit der Öffnung nach oben bzw. das einführseitige Ende nach unten im wesentlichen senkrecht gehalten werden.
Die Pille kann durch einfaches fallen lassen in die Einführhülse eingebracht werden, sie richtet sich in der Spitze der Einführhülse selbständig aus. Danach kann der Tampon eingesteckt und die Ausstosshülse eingesetzt werden. Dieser Vorgang kann durch einfach aufgebaute Maschinen schnell ausgeführt werden.

Indem an der Einführhülse Haltemittel angebracht sind, kann die Pille im in die Einführhülse eingesetzten Zustand gehalten werden, wodurch erreicht wird, dass die Pille beim Einsetzen des Tampons in die Einführhülse, auch wenn diese hierzu bewegt werden muss, am vorbestimmten Ort bleibt.

Eine vorteilhafte Ausgestaltung der Erfindung besteht darin, dass die Einführhülse und die Ausstosshülse jeweils aus einem rohrförmigen Körper aus Karton oder einem kartonähnlichen Stoff gebildet sind, der mindestens einseitig beschichtet ist, wodurch eine einfache und kostengünstige Herstellung erreicht werden kann. Vorteilhafterweise ist das einführseitige Ende der Einführhülse mit einem kalottenartigen Abschlussteil versehen, welcher durch zungenförmige Abschnitte gebildet ist, die jeweils im wesentlichen die Form eines Kugeldreiecks haben, und welche beim Ausstossen des Tampons zusammen mit der Pille aufspreizbar sind. Dadurch lässt sich dieser Tampon-Applikator problemlos in die Vagina einführen, das Ausstossen des Tampons zusammen mit der Pille wird problemlos erreicht.

Wenn eine Pille verwendet wird, deren beide Flächen die Form einer Kalotte aufweisen und demzufolge symmetrisch ist, ist das Einsetzen dieser Pille in die Einführhülse besonders einfach, da die Pille jeder beliebigen Lage in die Einführhülse eingebracht werden kann und darin ausgerichtet wird. Besonders einfach wird das Einsetzen der Pille in die Einführhülse, wenn die Pille die Form einer Kugel aufweist.

In vorteilhafter Weise ist die gegen die Spitze des Tampons gerichtete Fläche der Pille als konkave Fläche ausgebildet, die im wesentlichen mit der Form der Spitze des Tampons übereinstimmt. Ein üblicherweise verwendbarer Tampon muss nicht verändert werden. Dadurch wird ein optimaler Sitz der Pille auf dem Tampon erreicht, ein Einführen des Tampons zusammen mit der Pille ist problemlos möglich.

Eine weitere Ausführungsform kann einen Tampon mit einer Einbuchtung aufweisen, die im Bereich der Spitze des Tampons angebracht ist. Durch die Verwendung einer Pille, deren beide Flächen die Form einer Kalotte haben, wird die Pille in optimaler Weise im Tampon-Applikator untergebracht. Ein Einführen des Tampons zusammen mit der Pille in die Vagina ist in optimaler Weise möglich.

In vorteilhafter Weise ist jeweils der mit einem Tampon und der Pille versehene Tampon-Applikator mit einer luftdichten Verhüllung ausgestaltet, die vor der Anwendung des Tampon-Applikators aufreissbar und wegnehmbar ist. Dadurch werden die Anforderungen an die Hygiene erfüllt, die Pille verbleibt über eine längere Zeit im gewünschten Zustand.

Ausführungsbeispiele der vorliegenden Erfindung werden nachfolgend anhand der beiliegenden Zeichnung beispielhaft näher erläutert.

Es zeigt
Fig. 1 eine Ansicht einer ersten Ausführungsform eines erfindungsgemässen Tampon-Applikators im ursprünglichen Zustand, zum Teil im Schnitt;
Fig. 2 eine Ansicht des Tampon-Applikators gemäss Fig. 1 während des Ausstossens des Tampons zusammen mit der eingesetzten Pille, zum Teil im Schnitt; und
Fig. 3 bis 5 weitere Ausgestaltungsformen der Pille und deren Anordnung bezüglich des Tampons in der Einführhülse.

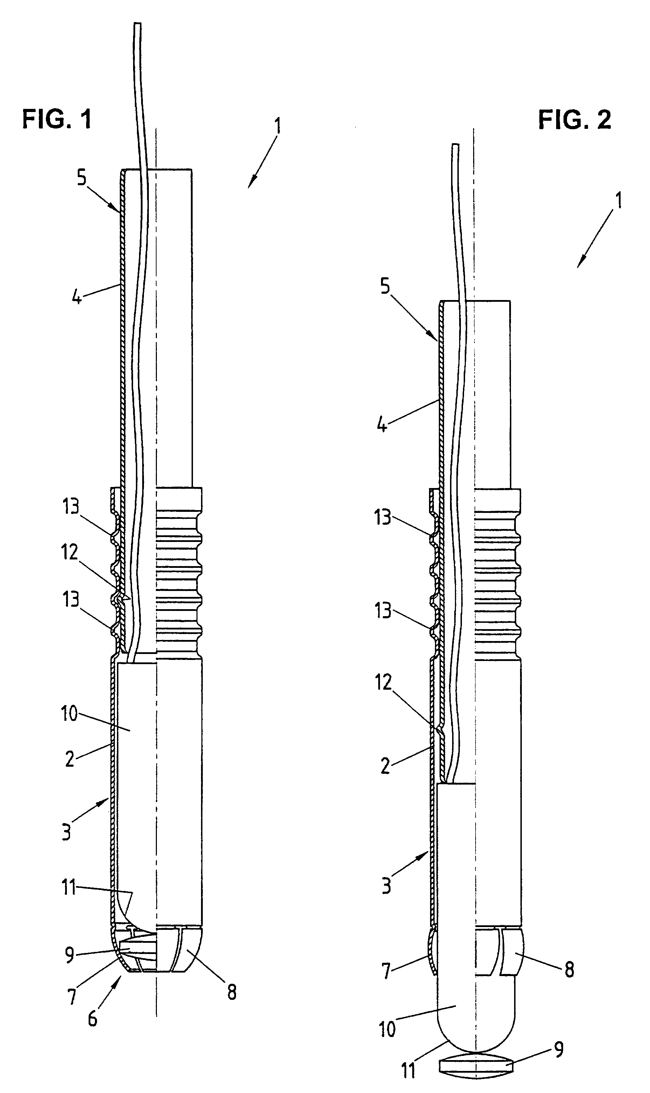

Wie aus Fig. 1 ersichtlich ist, besteht der erfindungsgemässe Tampon-Applikator 1 aus einer Einführhülse 2, die aus einem rohrförmigen Körper 3 gebildet ist, in welchem eine Ausstosshülse 4, die ebenfalls aus einem rohrförmigen Körper 5 gebildet ist, verschiebbar eingesetzt ist.

Das einführseitige Ende 6 der Einführhülse 2 ist mit einem kalottenartigen Abschlussteil 7 versehen, welcher durch zungenförmige Abschnitte 8 gebildet ist. Diese zungenförmige Abschnitte 8 weisen jeweils im wesentlichen die Form eines Kugeldreiecks auf und sind aufspreizbar, wie später noch beschrieben wird.

In die Einführhülse 2 ist eine Pille 9 eingelegt, die in den inneren Bereich des kalottenförmigen Abschlussteils 7 der Einführhülse 2 zu liegen kommt. Des weiteren ist in die Einführhülse 2 ein Tampon 10 eingesetzt, so dass die Pille 9 zwischen der Spitze 11 des Tampons 10 und der Innenseite des kalottenförmigen Abschlussteils 7 in der Einführhülse 2 gehalten ist. Vorteilhafterweise weist die Pille 9 eine konvex gekrümmte Fläche auf, die im wesentlichen an den kalottenartigen Abschlussteil 7 angepasst und an diesen anliegend ist. Die gegen die Spitze 11 des Tampons 10 gerichtete Fläche ist in diesem Ausführungsbeispiel entsprechend der gegenüberliegenden Fläche ebenfalls konvex ausgebildet.

Die Ausstosshülse 4 ist so weit in die Einführhülse 2 eingeschoben, dass sie mit ihrem inneren Endbereich an den Tampon 10 anstösst, wobei zum Festhalten dieser Lage die Ausstosshülse 4 mit einem Noppen 12 versehen ist, der in eine der entsprechend an der Einführhülse 2 angebrachten Rillen 13 einschnappen kann.

Die Einführhülse 2 und die Ausstosshülse 4 sind aus einem biegsamen Material gefertigt, beispielsweise Karton. Dieser Karton wird mit einer Schicht versehen, die mindestens eine gewisse Wasserfestigkeit aufweist.
Diese Schicht kann innenseitig an der Ausstosshülse 4 weggelassen werden. Selbstverständlich sind auch andere geeignete Materialien denkbar, beispielsweise auf Kunststoffbasis.

In diesem Zustand wird der Tampon-Applikator 1 in bekannter Weise in einer nicht dargestellten, luftdichten Verhüllung verpackt.

Vor der Anwendung dieses Tampon-Applikators 1 wird die Verhüllung weggenommen, der Tampon-Applikator 1 kann in die Vagina eingeführt werden. Die Ausstosshülse 4 wird dann in die Einführhülse 2 eingeschoben.
Der Tampon 10 drückt mit der Pille 9 gegen die zungenförmigen Abschnitte 8, die aufgespreizt werden, was durch die kalottenartige Form der entsprechenden Fläche der Pille optimal ermöglicht wird. Der Tampon 10 wird mit der Pille 9 aus der Einführhülse ausgestossen, wie dies in Fig. 2 dargestellt ist.

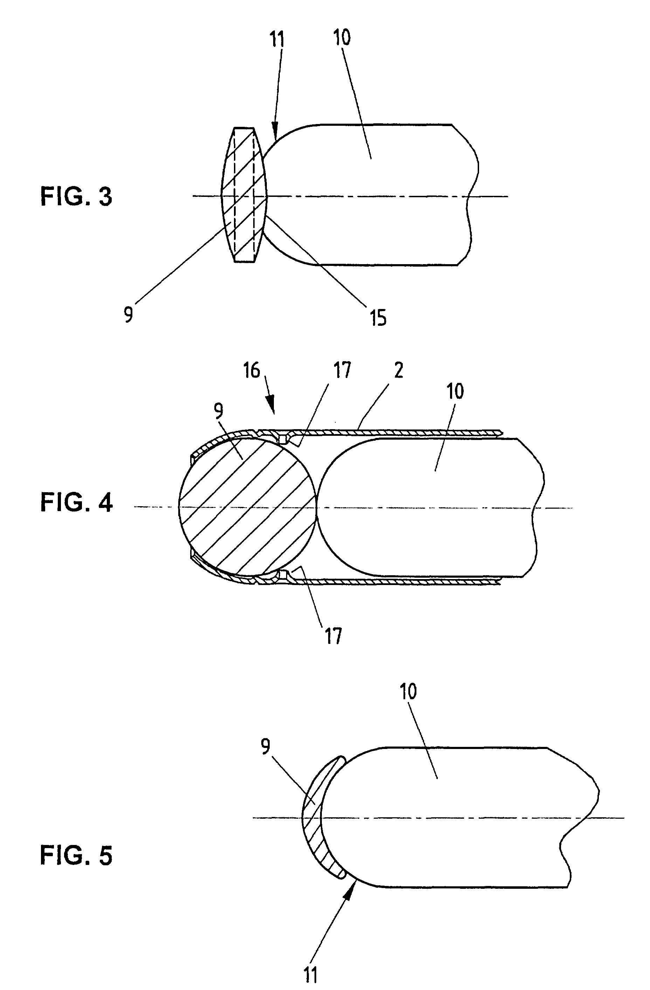

Wie in Fig. 3 dargestellt ist, kann in der Spitze 11 des Tampons 10 eine Einbuchtung 15 angebracht sein. In diese Einbuchtung 15 kommt die konvexe, gegen die Spitze 11 des Tampons 10 gerichtete Fläche der Pille 9 zu liegen, wobei diese Pille 9 gleich ausgestaltet ist, wie die in den Fig. 1 und 2 dargestellte Pille 9.

Aus Fig. 4 ist ersichtlich, dass die in den Fig. 1 und 2 dargestellten Pillen 9 auch die Form einer Kugel aufweisen können. In dem hier im Schnitt dargestellten vorderen Bereich der Einführhülse 2 sind Haltemittel 16 sichtbar, welche die in die Einführhülse 2 eingesetzte Pille 9 in der vorbestimmten Lage halten. Im dargestellten Beispiel bestehen die Haltemittel 16 aus zwei Einprägungen 17, die innenseitig vorstehen. Dadurch kann die Einführhülse 2 vor oder während des Einsetzens der Pille bewegt werden, ohne dass die Pille 9 ihre Lage ändert. Selbstverständlich können derartige Haltemittel 16 auch bei der Verwendung von Pillen 9 mit anderen Formen, beispielsweise bei den in den anderen Figuren dargestellten, angebracht werden.

Denkbar ist hier auch, dass der Tampon 10 mit einer Einbuchtung versehen werden könnte, die etwa derjenigen entspricht, die in Fig. 3 dargestellt ist.

Fig. 5 zeigt die Spitze 11 des Tampons 10, auf welche die Pille 9 aufgesetzt ist. Die der Spitze 11 des Tampons zugewandte Fläche ist entsprechend konkav ausgebildet, während die gegenüberliegende Fläche, die gegen das einführseitige Ende des Tampon-Applikators gerichtet ist, die Form einer Kalotte aufweist. Diese Pille 9 liegt somit beim Ausstossen des Tampons kappenartig auf der Spitze 11 des Tampons 10.

Mit diesen Ausführungsformen ist gesichert, dass die jeweilige Pille 9 zusammen mit dem Tampon 10 in optimaler Weise in der Vagina positioniert werden kann und sich die medizinische Wirkung entfalten kann.

## Patentansprüche

1. Tampon-Applikator (1) mit einer Einführhülse (2), in welcher ein Tampon (10) eingesetzt ist, mit einer innerhalb der Einführhülse (2) verschiebbaren Ausstosshülse (3) zum Ausstossen des Tampons (10), und mit einem medikamentösen Mittel, das die Form einer Pille (9) aufweist und zusammen mit dem Tampon (10) durch das einführseitige Ende (6) der Einführhülse (2) ausstossbar ist, **dadurch gekennzeichnet, dass** die Pille (9) derart in die Einführhülse (2) eingelegt ist, dass sie zwischen die Spitze (11) des Tampons (10) und das einführseitige Ende (6) der Einführhülse (2) zu liegen kommt und dass an der Einführhülse (2) Haltemittel (16) für die Pille (6) angebracht sind, die aus mindestens einer in der Einführhülse (2) angebrachten, nach innen vorstehenden Einprägung (17) bestehen, mit welcher die Pille (6) im in die Einführhülse (2) eingelegten Zustand gehalten ist.

2. Tampon-Applikator (1) nach Anspruch 1, **dadurch gekennzeichnet, dass** die Einführhülse (2) und die Ausstosshülse (3) jeweils aus einem rohrförmigen Körper (3, 5) aus Karton oder einem kartonähnlichen Stoff gebildet sind, der mindestens einseitig beschichtet ist, und dass das einführseitige Ende (6) der Einführhülse (2) mit einem kalottenartigen Abschlussteil (7) versehen ist, welcher durch zungenförmige Abschnitte (8) gebildet ist, die jeweils im wesentlichen die Form eines Kugeldreiecks haben, und welche beim Ausstossen des Tampons (10) zusammen mit der Pille (9) aufspreizbar sind.

3. Tampon-Applikator (1) nach Anspruch 1 oder 2, **dadurch gekennzeichnet, dass** mindestens die gegen das einführseitige Ende (6) des Tampons (10) gerichtete Fläche der Pille (9) die Form einer Kalotte aufweist.

4. Tampon-Applikator (1) nach Anspruch 3, **dadurch gekennzeichnet, dass** die gegen das einführseitige Ende (6) der Einführhülse (2) gerichtete Fläche und die gegen die Spitze (11) des Tampons (10) gerichtete Fläche der Pille (9) jeweils die Form einer Kalotte aufweist.

5. Tampon-Applikator (1) nach Anspruch 3 oder 4, **dadurch gekennzeichnet, dass** die Pille (9) die Form einer Kugel aufweist.

6. Tampon-Applikator (1) nach Anspruch 3, **dadurch gekennzeichnet, dass** die gegen die Spitze (11) des Tampons (10) gerichtete Fläche der Pille (9) eine konkave Form aufweist und im wesentlichen mit der Form der Spitze (11) des Tampons (10) übereinstimmt.

7. Tampon-Applikator (1) nach Anspruch 4 oder 5, **dadurch gekennzeichnet, dass** im Bereich der Spitze (11) des Tampons (10) eine Einbuchtung (15) angebracht ist, die der Form der Kalotte der Pille (9) entspricht.

8. Tampon-Applikator (1) nach einem der Ansprüche 1 bis 7, **dadurch gekennzeichnet, dass** die Pille (6) durch Pressen eines pulverförmigen Präparates gebildet ist.

9. Tampon-Applikator (1) nach einem der Ansprüche 1 bis 8, **dadurch gekennzeichnet, dass** der Tampon-Applikator (1) mit einer luftdichten Verhüllung versehen ist, die vor der Anwendung des Tampon-Applikators (1) aufreissbar und wegnehmbar ist.

## Claims

1. Tampon applicator (1) having an insertion sleeve (2) in which a tampon (10) is introduced, having an ejection sleeve (3) slidable within the insertion sleeve (2) for ejecting the tampon (10), and having a medicinal agent, which has the form of a pill (9) and is ejectable together with the tampon (10) through the leading end (6) of the insertion sleeve (2), **characterized in that** the pill (9) is placed in the insertion sleeve (2) in such a way that it comes to lie between the tip (11) of the tampon (10) and the leading end (6) of the insertion sleeve (2) and **in that** provided on the insertion sleeve (2) are holding means (16) for the pill (6) <sic.(9)> which consist of at least one inwardly protruding impression (17) made in the insertion sleeve (2) by means of which the pill (6) <sic. (9)> is held in the state placed in the insertion sleeve (2).

2. Tampon applicator (1) according to claim 1, **characterized in that** the insertion sleeve (2) and the ejection sleeve (3) are each formed of a tubular body (3, 5) of cardboard or a cardboard-like material which is coated on at least one side, and that the leading end (6) of the insertion sleeve (2) is provided with a cap-like closure portion (7) formed by tongue-shaped sections (8) which each have substantially the shape of a spherical wedge and which are expandable upon ejection of the tampon (10) together with the pill (9).

3. Tampon applicator (1) according to claim 1 or 2, **characterized in that** at least the surface of the pill (9) directed toward the leading end (6) of the tampon (10) has the shape of a cap.

4. Tampon applicator (1) according to claim 3, **characterized in that** the surface of the pill (9) directed toward the leading end (6) of the insertion sleeve (2) and the surface directed toward the tip (11) of the tampon (10) each have the shape of a cap.

5. Tampon applicator (1) according to claim 3 or 4, **characterized in that** the pill (9) has the shape of a sphere.

6. Tampon applicator (1) according to claim 3, **characterized in that** the surface of the pill (9) directed toward the tip (11) of the tampon (10) has a concave shape and substantially coincides with the shape of the tip (11) of the tampon (10).

7. Tampon applicator (1) according to claim 4 or 5, **characterized in that** made in the area of the tip (11) of the tampon (10) is a recess (15) which corresponds to the shape of the cap of the pill (9).

8. Tampon applicator (1) according to one of the claims 1 to 7, **characterized in that** the pill (6) [sic] is formed by pressing of a powdery preparation.

9. Tampon applicator (1) according to one of the claims 1 to 8, **characterized in that** the tampon applicator (1) is provided with a hermetic cover which can be tom open and removed prior to use of the tampon applicator (1).

## Revendications

1. Applicateur de tampon (1) avec un manchon d'introduction (2) dans lequel est introduit un tampon (10) avec un manchon d'expulsion (3) coulissant à l'intérieur du manchon d'insertion (2) pour expulser le tampon (10) et un agent médicamenteux qui présente la forme d'une pastille (9) et qui peut être expulsée ensemble avec le tampon (10) par l'extrémité (6) d'introduction du manchon d'introduction (2), **caractérisé en ce que** la pastille (9) est insérée de telle manière dans le manchon d'introduction (2) qu'elle vient s'appliquer entre la pointe (11) du tampon (10) et l'extrémité d'introduction (6) du manchon d'introduction (2) et **en ce qu'**il est ménagé sur le manchon d'introduction (2) des moyens de retenue (16) pour la pastille (6) qui se composent d'au moins une empreinte creuse (17) ménagée dans le manchon d'introduction (2) qui permet de maintenir la pastille (6) dans l'état introduit dans le manchon d'introduction (2).

2. Applicateur de tampon (1) selon la revendication 1, **caractérisé en ce que** le manchon d'introduction (2) et le manchon d'expulsion (3) sont respectivement formés par un corps tubulaire (3, 5) en carton ou en matériau cartonné similaire qui est au moins enduit sur un côté et **en ce que** l'extrémité côté introduction (6) du manchon d'introduction (2) est munie d'une partie couvercle en forme de calotte (7) qui est formée par des sections en forme de languette (8) qui ont sensiblement la forme d'un triangle sphérique et qui peuvent être écartées lors de l'expulsion connue du tampon (10) et de la pastille (9).

3. Applicateur de tampon (1) selon la revendication 1 ou 2, **caractérisé en ce qu'**au moins la surface de la pastille (9), qui est dirigée en direction de l'extrémité côté introduction (9) du tampon (10) présente la forme d'une calotte.

4. Applicateur de tampon (1) selon la revendication 3, **caractérisé en ce que** la surface de la pastille (9), qui est dirigée en direction de l'extrémité côté introduction (6) du manchon d'introduction (2) et la surface de la pastille (9) dirigée vers la pointe (11) du tampon (10) présentent respectivement la forme d'une calotte.

5. Applicateur de tampon (1) selon la revendication 3 ou 4, **caractérisé en ce que** la pastille (9) présente la forme d'une sphère.

6. Applicateur de tampon (1) selon la revendication 3, **caractérisé en ce que** la surface de la pastille (9) dirigée vers la pointe (11) du tampon (10) présente une forme concave et coïncide essentiellement avec la forme de la pointe (11) du tampon (10).

7. Applicateur de tampon (1) selon la revendication 4 ou 5, **caractérisé en ce que** dans la zone de la pointe (11) du tampon (10), il est ménagé un renfoncement (15) qui correspond à la forme de la calotte de la pastille (9).

8. Applicateur de tampon (1) selon l'une des revendications 1 à 7, **caractérisé en ce que** la pastille (6) est formée par pressage d'une préparation pulvérulente.

9. Applicateur de tampon (1) selon l'une des revendications 1 à 8, **caractérisé en ce que** l'applicateur de tampon (1) est muni d'une enveloppe étanche à l'air qui peut être retirée et arrachée avant l'utilisation de l'applicateur de tampon (1).
